Europäisches Patentamt

European Patent Office

Office européen des brevets

(11) Publication number: **0 443 740 A2**

## EUROPEAN PATENT APPLICATION

(21) Application number: **91300900.7**

(51) Int. Cl.5: **A61K 7/09, A61K 7/06**

(22) Date of filing: **04.02.91**

(30) Priority: **22.02.90 US 483367**

(43) Date of publication of application:
**28.08.91 Bulletin 91/35**

(84) Designated Contracting States:
**AT BE CH DE ES FR GB IT LI LU NL SE**

(71) Applicant: **REVLON, INC.**
**625 Madison Avenue**
**New York, NY 10022(US)**

(72) Inventor: **Klein, Gustave J.**
**5 Garden Street, Great Neck**
**New York 11021(US)**
Inventor: **Cot, Jaime Ventos**
**Sabino De Arana, 46,50,2a**
**08028 Barcelona(ES)**

(74) Representative: **W.P. THOMPSON & CO.**
**High Holborn House, 52-54 High Holborn**
**London WC1V 6RY(GB)**

(54) Permanent hair waving kit and process.

(57) A method for providing permanent waves under constant time and temperature conditions while varying the concentration, pH and activity rate of the reducing agent in accordance with the specific conditions of the hair. The method comprises the evaluation of the hair into one of 972 sub-classes on the basis of its physical properties. A characteristic permanent wave formula is mixed from a plurality of stock mixture in accordance with this evaluation. All such formulae are applied at constant conditions of time and temperature to the differing type of hair. This method may be provided as a kit consisting of containers for the storage, mixing and application of stock mixtures in addition to means for evaluation of hair type and condition.

EP 0 443 740 A2

## Background of the Invention

### Field of the Invention

This invention relates to cosmetic lotions. It particularly relates to a kit and process useful for making "tailor-made" lotions, such as a permanent wave, by mixing base solutions at the point-of-sale based on the individual needs of the person.

### Description of the Prior Art

In the permanent waving of hair, the hair is treated with a reductant, such as ammonium thioglycolate, to reduce (break) the disulfide linkages in the hair proteins to sulfhydryl groups. This breakage in the disulfide linkages diminishes the rigidity of the hair proteins, leaving the hair more pliable. The hair is then set, on curlers, rods or rollers. If preferred, the hair may be set prior to reduction. The set hair is then treated with an oxidant, such as a peroxide, which oxidizes the sulfhydryl groups to disulfide linkages thereby imparting a rigidity to the wave obtained by setting. The "perfect perm" should reduce the number of disulfide bonds in the hair necessary to give a permanent change in shape, but not more than necessary. Excess reduction causes weak, dry, or "breaking" hair. Under reduction will cause "temporary" permanent wave results. Lotions for permanent waving contain from about 3 to 12% by weight of ammonium thioglycolate and an alkali, usually ammonium hydroxide, to maintain the pH at about 8.5 to 10.5. Lotions having a higher pH or a greater concentration of the reductant are more active than those having a lower pH or lower concentration.

U.S. Patent No. 4,158,704 discloses a process and composition for the permanent waving of hair in a beauty shop which employs certain esters of thioglycolic acid as reducing agents. The process and composition, however, is employed at elevated temperatures of about 80° to 130° F (about 27° to 55° C) and at a pH of about 6.4 to 8.3. The hair being treated is pre-evaluated in accordance with a specific criteria. The hair is first characterized into four types of hair, i.e., Bleached, Tinted, Normal or Resistant. Then, with respect to the respective primary criteria for the hair, the hair is further subclassified according to porosity as being either "highly to slightly" or "very slightly to none" or "none." Thus, the hair is subclassified into one of twelve (12) possible subclassifications depending on the condition of the hair relative to both the primary and secondary criteria used to evaluate it. Based on the resulting characterization of the hair, the hair is then treated with one lotion having a concentration, pH and activity rate which is fixed. The lotion, however, is used under different temperature and time conditions depending on the type of hair being treated and its porosity.

While presenting a distinct improvement in the art by eliminating the need for subjective evaluation of the "test curl", the above patent still has the disadvantage of using a hair dryer. These dryers are expensive, place the client under heat, and are very difficult to control relative to the precise temperatures necessary to achieve a "perfect-perm." Moreover, the twelve (12) categories of hair under type and porosity are not nearly enough to cover all the variables presented by human hair. These variables include not only porosity and type of hair but also the additional variables of hair thickness or diameter, length of hair, and population or density. Still another disadvantage is the preformulation of the lotion at the factory prior to its use. In fact, all prior art lotions, including those for permanent waves, are preformulated and adapted to be stored for extended periods of time as distinguished from being mixed from a plurality of base mixtures at the point-of-sale. The reductant is very expensive, extremely reactive and has a strong odor. Any unused portion of the lotion which is left in the bottle once opened is discarded.

The prior art has prepared at least four (4) specific lotions in varying strengths for hard-to-wave, normal, easy-to-wave and for resistant hair. See in this regard Cosmetics: Science And Technology, 1972, Volume 2, pages 206-207. However, these preparations have been limited to fixed formulations (mixed and bottled at the factory) that either overprocess or underprocess the hair due to the many variables involved and the subjective judgment of the operator.

Another disadvantage of the prior art preformulated cosmetics and hair lotions is the fact that these products must be shelf-stable. Ingredients which react or degrade when mixed together and stored for any length of time cannot be used. Many hair conditioners, buffers, and other hair treatment chemicals are sensitive to the reducing agent and cannot be used due to a very short shelf-life. For example, hydrolyzed protein derivatives when used in hair wave formulations react with the reductant to produce ammonia and carbon dioxide as degradation products. Cationic molecules, such as the polyquaternium conditioners, react with the alkaline reductant. Many anhydrous ingredients that would be useful cannot be mixed with water since they hydrolyze after a few hours. Guanidine hydroxide, a good relaxer, is not stable and must be supplied in two (2) parts, one containing calcium hydroxide and the other, guanidine carbonate, and then mixed together at the time of use. Moreover, controlled exothermic reactions cannot be achieved without the separation of oxidizing agent (hydrogen peroxide) from the reducing agent. Thus, "hot" products are not possible without the mixing of these chemicals at the point-of-sale by the operator.

It would thus be desirable to reduce the variables inherent in the above prior art products by having several stock mixtures which can be provided in separate containers and adapted to be mixed or combined at the point-of-use to provide a tailor-made cosmetic based on the specific needs of the individual. The use of separate stock mixtures which are separated would allow the use of chemically active ingredients which presently cannot be used due to shelf-stability problems.

## Summary of the Invention

It is accordingly an object of this invention to provide a plurality of base or stock cosmetic mixtures adapted to be blended or mixed at the point-of-use to fit the individual needs of the client.

Another object of this invention is to mix at least twelve (12) and as many as thirty-six (36) or more different lotions at the point-of-use based on the specific needs of the client.

Another object of this invention is to mix chemically active ingredients at the point-of-use from stock mixtures which are separated in order to avoid shelf-stability problems or to provide controlled exothermic reactions, such as a "hot" lotion.

Another object of the present invention is to provide a process for permanent waving which will eliminate the subjective judgment of the operator in determining the conditions for waving.

Another object of the present invention is to provide a process for use in permanent waving in which the time and temperature are constant and the reducing treatment is controlled objectively by varying the formula.

It is still another object of the present invention to provide a reducing composition for use in permanent waving, which can be used on untreated hair, whether resistant or normal, or hair that has been previously permed, bleached or dyed.

This invention, accordingly, involves the mixing of tailor-made cosmetic lotions at the point-of-use in accordance with the varied criteria of the client to be treated and comprises providing a plurality of stock cosmetic mixtures, providing a plurality of sets of criteria based on differing cosmetic requirements, one set of criteria for each lotion to be mixed, selecting the set of criteria desired, mixing the cosmetic lotion in accordance with said set of criteria, and then applying the tailor-made lotion to the client within 30 minutes.

More particularly, in accordance with this invention, there is provided a process for effecting the reducing step in permanent waving under constant time and temperature conditions while varying the concentration and pH of the reducing agent in accordance with the conditions of the hair. This comprises providing a plurality of primary stock solutions or mixtures; providing a plurality of sets of criteria based on the type and condition of the hair for varying the concentration, pH, and amount of reducing agent to be mixed from said plurality of primary mixtures, one set of criteria for each final solution mixed; evaluating the hair to choose said set of criteria; mixing a final mixture of permanent wave in accordance with the chosen criteria; applying the permanent wave to the hair; and keeping the time and temperature constant for the application of each permanent wave to differing types and conditions of hair.

Regardless of the type and condition of the hair, the permanent wave of this invention is carried out at $30° \pm 1°$ in all cases. This is the normal scalp temperature and is very precise in all cases. This temperature is assured by placing a cap over the hair during the permanent wave. Similarly, the time is kept constant in all cases at 18 minutes $\pm$ 5 minutes. By keeping the time and temperature the same in all cases while also subdividing the hair in accordance with at least thirty-six (36) specific permanent wave mixtures, the danger of over-processing or under-processing is avoided.

A preferred feature of this point-of-use mixing of the stock mixtures of this invention is that chemically sensitive or time-sensitive ingredients which normally cannot be used when mixed at the factory can now be used immediately without encountering the shelf-stability problems inherent in such compositions.

## Detailed Description of the Invention

### Process of the Present Invention

In order to carry out the process of the present invention, the hair being treated is first characterized by the beauty parlor operator as falling within one of four (4) general classifications or basic types of hair depending on its degree of damage. These four basic types are Bleached, Tinted, Normal and Resistant.

"Bleached" represents hair that is most damaged while "Resistant" represents a hair type that is least damaged. "Normal" means average hair that has not been damaged by chemicals while "Tinted" represents slightly damaged hair. This nomenclature is understood by the hair stylist but any other similar nomenclature can be used.

After being placed in one of such four primary classes of hair, the hair to be treated is then further subclassified by the beauty parlor operator into one of three (3) secondary classifications of hair based on porosity. These secondary classifications indicate that the hair is either highly porous, medium, or low in porosity. Porosity is an important factor since it indicates the ability of the hair to absorb fluid, i.e., the permanent wave. The professional judgment of the hair stylist determines the selection of basic hair type (Bleached, Tinted, Normal and Resistant) and porosity (High, Medium, and Low), and the stylist may additionally take into consideration other recent treatments to the person's hair.

Hair falling within each of the four basic classifications will also be subclassifiable in each of the secondary classifications. Hair thus first falling into one of such primary subclasses of hair, and then into one of such secondary subclasses, falls automatically into one of twelve (12) subclasses.

After being placed in one of such three secondary subclasses of hair, the hair is further subclassified by diameter or thickness of the hair into another tertiary group of classification criteria, to indicate whether the hair is of fine, medium or coarse diameter.

The subclassification of each client's hair in this manner enables the beauty parlor operator to obtain a much finer description of the condition of the client's hair at any one given time. It thus enables the beauty parlor operator to provide more reproducible results from one permanent wave treatment to the next, since it takes into consideration the more basic condition of the client's hair.

Hair falling within one of the four basic classifications and one of the secondary classes will also be sub-subclassifiable in each of the tertiary subclasses and thus falls, automatically, into one of thirty six (36) sub-subclasses of hair. After the hair is sub-subclassified into one of the thirty six (36) subclasses of hair, it is then treated with a specific hair waving mixture for such subclass of hair as provided by Table I as disclosed below.

TABLE I

Permanent Wave Mixture Varying As To Concentration & pH (% By Weight)

**Medium**

| | 1 | 2 | 3 | 4 | 5 | 6 | 7 | 8 | 9 | 10 | 11 | 12 |
|---|---|---|---|---|---|---|---|---|---|---|---|---|
| Ammonium thioglycolate (50%) | 22.0 | 21.0 | 20.0 | 19.0 | 18.0 | 17.0 | 16.0 | 15.0 | 13.5 | 12.0 | 11.0 | 10.4 |
| Diammonium dithioglycolate | 3.0 | 4.1 | 5.1 | 6.2 | 7.2 | 8.3 | 9.3 | 10.4 | 10.9 | 11.5 | 12.5 | 12.9 |
| Ammonium hydroxide (25%) | 5.0 | 4.6 | 4.2 | 3.8 | 3.4 | 3.0 | 2.6 | 2.2 | 1.8 | 1.4 | 1.0 | 0.7 |
| Polyquaternium - 4 | - | - | - | - | - | - | - | - | - | 1.1 | 1.2 | 1.4 |
| Polyquaternium - 10 | 1.0 | 1.1 | 1.2 | 1.3 | 1.4 | 1.5 | 1.6 | 1.7 | 1.8 | 1.0 | 2.0 | 2.1 |
| Na$_5$ (EDTA) | 0.2 | 0.2 | 0.2 | 0.2 | 0.2 | 0.2 | 0.2 | 0.2 | 0.2 | 0.2 | 0.2 | 0.2 |
| Propylene glycol | 6.0 | 6.0 | 6.0 | 6.0 | 6.0 | 6.0 | 6.0 | 6.0 | 6.0 | 6.0 | 6.0 | 6.0 |
| Laureth 23 | 2.0 | 2.0 | 2.0 | 2.0 | 2.0 | 2.0 | 2.0 | 2.0 | 2.0 | 2.0 | 2.0 | 2.0 |
| Perfume | 0.5 | 0.5 | 0.5 | 0.5 | 0.5 | 0.5 | 0.5 | 0.5 | 0.5 | 0.5 | 0.5 | 0.5 |
| Water | 60.3 | 60.5 | 60.8 | 61.0 | 61.3 | 61.5 | 61.8 | 62.0 | 62.3 | 63.4 | 63.6 | 63.8 |
| pH | 9.9 | 9.8 | 9.7 | 9.5 | 9.3 | 9.1 | 8.9 | 8.7 | 8.5 | 4 | 8.1 | 7.9 |

**Fine**

| | 13 | 14 | 15 | 16 | 17 | 18 | 19 | 20 | 21 | 22 | 23 | 24 |
|---|---|---|---|---|---|---|---|---|---|---|---|---|
| Ammonium thioglycolate (50%) | 22.0 | 21.0 | 20.0 | 19.0 | 18.0 | 17.0 | 16.0 | 15.0 | 13.5 | 12.0 | 11.0 | 10.4 |
| Diammonium dithioglycolate | 3.0 | 4.1 | 5.1 | 6.2 | 7.2 | 8.3 | 9.3 | 10.4 | 10.9 | 11.5 | 12.5 | 12.9 |
| Ammonium hydroxide (25%) | 5.0 | 4.6 | 4.2 | 3.8 | 3.4 | 3.0 | 2.6 | 2.2 | 1.8 | 1.4 | 1.0 | 0.7 |
| Polyquaternium - 4 | - | - | - | - | - | - | - | - | - | 1.1 | 1.2 | 1.4 |
| Polyquaternium - 10 | 1.0 | 1.1 | 1.2 | 1.3 | 1.4 | 1.5 | 1.6 | 1.7 | 1.8 | 1.0 | 2.0 | 2.1 |
| Na$_5$ (EDTA) | 0.2 | 0.2 | 0.2 | 0.2 | 0.2 | 0.2 | 0.2 | 0.2 | 0.2 | 0.2 | 0.2 | 0.2 |
| Propylene glycol | 6.0 | 6.0 | 6.0 | 6.0 | 6.0 | 6.0 | 6.0 | 6.0 | 6.0 | 6.0 | 6.0 | 6.0 |
| Laureth 23 | 2.0 | 2.0 | 2.0 | 2.0 | 2.0 | 2.0 | 2.0 | 2.0 | 2.0 | 2.0 | 2.0 | 2.0 |
| Perfume | 0.5 | 0.5 | 0.5 | 0.5 | 0.5 | 0.5 | 0.5 | 0.5 | 0.5 | 0.5 | 0.5 | 0.5 |
| Water | 55.3 | 55.5 | 55.8 | 56.0 | 56.3 | 56.5 | 56.8 | 57.0 | 57.3 | 58.4 | 58.6 | 58.8 |
| pH | 9.9 | 9.8 | 9.7 | 9.5 | 9.3 | 9.1 | 8.9 | 8.7 | 8.5 | 4 | 8.1 | 7.9 |

**Coarse**

| | 25 | 26 | 27 | 28 | 29 | 30 | 31 | 32 | 33 | 34 | 35 | 36 |
|---|---|---|---|---|---|---|---|---|---|---|---|---|
| Ammonium thioglycolate (50%) | 22.0 | 21.0 | 20.0 | 19.0 | 18.0 | 17.0 | 16.0 | 15.0 | 13.5 | 12.0 | 11.0 | 10.4 |
| Diammonium dithioglycolate | 3.0 | 4.1 | 5.1 | 6.2 | 7.2 | 8.3 | 9.3 | 10.4 | 10.9 | 11.5 | 12.5 | 12.9 |
| Ammonium hydroxide (25%) | 5.0 | 4.6 | 4.2 | 3.8 | 3.4 | 3.0 | 2.6 | 2.2 | 1.8 | 1.4 | 1.0 | 0.7 |
| Polyquaternium - 4 | - | - | - | - | - | - | - | - | - | 1.1 | 1.2 | 1.4 |
| Polyquaternium - 10 | 1.0 | 1.1 | 1.2 | 1.3 | 1.4 | 1.5 | 1.6 | 1.7 | 1.8 | 1.0 | 2.0 | 2.1 |
| Na$_5$ (EDTA) | 0.2 | 0.2 | 0.2 | 0.2 | 0.2 | 0.2 | 0.2 | 0.2 | 0.2 | 0.2 | 0.2 | 0.2 |
| Propylene glycol | 6.0 | 6.0 | 6.0 | 6.0 | 6.0 | 6.0 | 6.0 | 6.0 | 6.0 | 6.0 | 6.0 | 6.0 |
| Laureth 23 | 2.0 | 2.0 | 2.0 | 2.0 | 2.0 | 2.0 | 2.0 | 2.0 | 2.0 | 2.0 | 2.0 | 2.0 |
| Perfume | 0.5 | 0.5 | 0.5 | 0.5 | 0.5 | 0.5 | 0.5 | 0.5 | 0.5 | 0.5 | 0.5 | 0.5 |
| Water | 65.3 | 65.5 | 65.8 | 66.0 | 66.3 | 66.5 | 66.8 | 67.0 | 67.3 | 68.4 | 68.6 | 68.8 |
| pH | 9.9 | 9.8 | 9.7 | 9.5 | 9.3 | 9.1 | 8.9 | 8.7 | 8.5 | 4 | 8.1 | 7.9 |

Each of the thirty-six (36) specific solutions of Table I are water based and comprise, in weight %,

about 3 to 20%, and preferably about 5 to 15%, of ammonium thioglycolate or other reducing agent,

about 2 to 20%, and preferably about 3 to 14%, of diammonium dithioglycolate or other suitable blocking agent or buffer,

about 0.5 to 3%, and preferably about 0.1 to 2%, ammonium hydroxide or other suitable alkali,

about 0.05 to 5%, and preferably about 0.1 to 3%, of one or more hair conditioners such as polyquaternium-4 and polyquaternium-10,

about 0.5 to 5%, and preferably about 1 to 3%, of one or more surfactants, such as Laureth 23,

about 0.1 to 0.3%, and preferably about 0.2%, of the penta sodium salt of diethylenetriaminepentaacetic acid or other suitable chelating agent,

about 3 to 10%, and preferably of about 5 to 7%, of propylene glycol, or other suitable humectant,

about 0.1 to 0.7%, and preferably about 0.3 to 0.7%, of one or more perfumes or fragrances, and

the balance water; about 50 to 75, and preferably about 55 to 70%.

The reducing agents which may be used would include ammonium thioglycolate (ATG), esters of thioglycolic acid, ammonium sulfites, and sodium hydroxide.

The blocking agents or buffers which may be used include diammonium dithioglycolate.

The alkalis which may be used would include ammonium hydroxide, monoethanolamine, diethanolamine or triethanolamine.

The hair conditioners which may be used would include all those useful for such purposes in hair waving solutions and would include all those listed in the CTFA Cosmetic Ingredient Handbook, at pages 71-73 thereof. Such hair conditioners would preferably include polyquaternium-4 (a copolymer of hydroxyethylcellulose and dialkyldimethyl ammonium chloride), and polyquaternium-10 (a polymeric quaternary ammonium salt of hydroxyethyl cellulose), and hydrolyzed protein derivatives.

The surfactants which may be used would include all those useful for such purposes in hair waving solutions and would include all those listed in the CTFA Cosmetic Ingredient Handbook, at pages 87-94 thereof (including those listed as cleansing agents and as emulsifying agents). Such surfactants would preferably include Laureth-23 (a polyethylene glycol ether of lauryl alcohol having the formula $CH_3(CH_2)_{10}$, $CH_2COCH_2CH_2)_n$ OH wherein n has a value of 23) and other nonionic polyethylene glycol ethers of cetyl alcohol and lauryl alcohol.

The colors and opacifiers would include all those useful in hair waving solutions and would include all those colors listed in the CTFA Cosmetic Ingredient Handbook at page 63 thereof. The opacifiers would include styrene-acrylate copolymer and all those opacifiers listed at page 75 thereof.

The listings of the hair conditioners, surfactants and opacifiers from the CTFA Cosmetic Ingredient Handbook are hereby incorporated herein by reference. The CTFA Cosmetic Ingredient Handbook in question is the 1st Edition, 1988, Editor J. M. Nikitakis.

TABLE II

| Primary Solution Formulation Component | % By Weight of Component in Container # | | | |
|---|---|---|---|---|
| | 1 | 2 | 3 | 4 |
| ammonium thioglycolate | 36.0 | 24.0 | 12.0 | 4.0 |
| diammonium dithioglycolate | -- | -- | -- | 28.0 |
| ammonium hydroxide | 15.6 | -- | -- | -- |
| Polyquaternium-4 | -- | -- | 2.0 | -- |
| Polyquaternium-10 | -- | -- | 2.5 | -- |
| $Na_5(EDTA)_5$ | 0.2 | 0.2 | 0.2 | 0.2 |
| propylene glycol | 6.0 | 6.0 | 6.0 | 6.0 |
| water | 7.0 | 7.0 | 7.0 | 7.0 |
| Laureth-23 | 2.0 | 2.0 | 2.0 | 2.0 |
| perfume | 0.5 | 0.5 | 0.5 | 0.5 |
| water | 32.1 | 60.1 | 66.8 | 52.0 |
| D&C yellow No. 10 0.5% solution | 0.6 | -- | -- | -- |
| FD&C blue No. 1 1.0% solution | -- | -- | -- | 0.3 |
| D&C red No. 33 1.0% solution | -- | -- | 1.0 | -- |
| styrene-acrylate copolymer | -- | 0.2 | -- | -- |
| pH | 14 | 7 | 7 | 7 |
| TOTAL | 100.00 | 100.00 | 100.00 | 100.00 |

The Table I mixtures or solutions of thirty-six (36) permanent wave formulations are mixed from the four (4) primary stock mixtures shown in Table II. While four (4) primary mixtures are shown, three (3), five (5), or more than five (5) may be used to achieve the point-of-use mixing. The stock mixtures employ 2 to 38% reductant (ammonium thioglycolate), 25 to 30% buffer (diammonium dithioglycolate), and 12 to 18% alkali (ammonium hydroxide). The hair conditioners (polyquaternium-4 and polyquaternium-10) are used at 1 to 3%. The stock mixtures can also contain a chelating agent such as $Na_5$ $(EDTA)_5$ at 0.1 to 0.3, a surfactant (Laureth 23) at 0.5 to 5%, and a humectant (propylene glycol) at 3-10% which also serves as a thickener or viscosity control agent.

Perfume, colors, fragrances, and opacifiers can be added at 0.1 to 1% with the remainder of the primary solutions being water which usually ranges from 30 to 60% by weight. The function of the colors and opacifiers is such that they can be employed in the stock mixtures in a fashion which will indicate an erroneous mixing of the final permanent wave.

The stock mixtures of Table II are so formulated (based on past customer usage by the beauty parlors) that they will exhaust together or closely together so that little or no waste of stock mixtures is encountered.

This is done by comparing the preset formulations of Table I against statistical averages of customer usage and then preparing the stock mixtures of Table II so that they can be subsequently blended to form said preset formulations in view of said statistical distribution such that each stock mixture is consumed at substantially the same rate.

After the hair is subclassified into one of thirty-six (36) subclasses corresponding to the thirty-six (36) specific formulas of Table I for each subclass of hair, it is possible with this invention to further classify the hair into twenty-seven (27) additional subclasses based on diameter (fine, medium, or coarse), length (short, medium, and long) and population or density (low, medium, and high). This subclassification will control the amount of quantity of formula to be dispensed based on the amount of hair to be treated.

Hair Waving Kit Of The Present Invention

To facilitate the use of the kit and process of the present invention to treat hair, as is described in more detail below, it is desirable to apply a code to each of the classes and subclasses of hair being treated. The resulting code can be of three digits. The first digit thereof could be one of the letters "R", "N", "T" and "B" which would stand for, respectively, Resistant, Normal, Tinted and Bleached. "Resistant" being the least damaged hair and Bleached the most damaged. The second digit of the code could be one of " L", "M" or "H" to stand for the degree of porosity (low, medium and high porosity). The third digit of the code could be one of the letters "F", "M" and "C", which would stand for the diameter of the hair (fine, medium and coarse).

This three digit code can then be used as a means to readily "pin-point" a hair sample into one of thirty six (36) specific permanent wave mixtures or solutions of Table I. Such a coding system which can be used to classify the hair is shown in Table II.

The hair waving process of the present invention is carried out with the aid of a kit that is provided for the use of the beauty parlor operator. This kit contains, preferably in pre-packaged form, all the chemicals needed to carry out the process. By using the prepackaged kit, the beauty parlor operator does not have to manually mix any chemicals. The kit comprises a series or set of about three (3) to five (5), preferably four (4), containers or reservoirs which, respectively, contain a series of three (3) to five (5), preferably four (4), stock mixtures. These stock mixtures contain graduated amounts of the reducing agent system to be used for the permanent wave treatment. Various amounts of each of these stock mixtures are then blended together to form a secondary mixture for the permanent wave. The composition of the secondary mixture will vary depending on the specific sub-subclass of hair to be treated. Since there are thirty six sub-subclasses of hair, according to the present invention, there will be provided, as noted above, a selection of thirty-six (36) secondary mixtures or specific formulas for use in the present invention. Each one of such thirty-six (36) secondary mixtures of permanent wave is thus tailored for use with only one of such thirty-six (36) sub-subclasses of hair.

## TABLE III

| Sub-subclass of Hair | Amount Of Stock Mixtures, In Grams, From Containers 1 To 4 Of Table II Used To Make A Desired Final Mixture For Permanent Wave | | | |
|---|---|---|---|---|
| | Container N = 1 | Container N = 2 | Container N = 3 | Container N = 4 |
| 1. RLF | 34 | 33 | 21 | 18 |
| 2. RLM | 34 | 32 | 22 | 18 |
| 3. RLC | 33 | 33 | 22 | 18 |
| 4. RMF | 32 | 32 | 21 | 21 |
| 5. RMM | 32 | 30 | 24 | 20 |
| 6. RMC | 31 | 29 | 24 | 22 |
| 7. RHF | 31 | 28 | 22 | 25 |
| 8. RHM | 30 | 27 | 24 | 25 |
| 9. RHC | 29 | 27 | 24 | 26 |
| 10. NLF | 28 | 26 | 24 | 28 |
| 11. NLM | 27 | 26 | 26 | 27 |
| 12. NLC | 26 | 26 | 27 | 27 |
| 13. NMF | 25 | 25 | 27 | 29 |
| 14. NMM | 24 | 25 | 28 | 29 |
| 15. NMC | 23 | 25 | 28 | 30 |
| 16. NHF | 22 | 24 | 28 | 32 |
| 17. NHM | 22 | 23 | 29 | 32 |
| 18. NHC | 21 | 23 | 29 | 33 |
| 19. TLF | 20 | 21 | 30 | 35 |
| 20. TLM | 19 | 20 | 32 | 35 |
| 21. TLC | 18 | 19 | 32 | 37 |
| 22. TMF | 18 | 18 | 32 | 38 |
| 23. TMM | 17 | 18 | 32 | 39 |
| 24. TMC | 16 | 17 | 32 | 41 |
| 25. THF | 15 | 16 | 33 | 42 |
| 26. THM | 14 | 15 | 34 | 43 |
| 27. THC | 13 | 15 | 35 | 43 |
| 28. BLF | 12 | 14 | 37 | 43 |
| 29. BLM | 12 | 13 | 37 | 44 |
| 30. BLC | 11 | 13 | 37 | 45 |
| 31. BMF | 10 | 13 | 38 | 45 |
| 32. BMM | 10 | 12 | 38 | 46 |
| 33. BMC | 10 | 11 | 38 | 47 |
| 34. BHF | 9 | 11 | 39 | 47 |
| 35. BHM | 9 | 10 | 40 | 47 |
| 36. BHC | 9 | 9 | 41 | 47 |

The stock mixtures disclosed in Table II are used to form thirty-six (36) secondary mixtures as they may be individually needed in order to treat each sub-subclass of hair. When the hair is sub-subclassified into one of the thirty-six (36) sub-subclasses of hair according to Table III, it is then treated with a final mixture therefor which is particularly designed to meet the permanent wave treatment needs of such sub-subclass of hair. In Table I, thirty-six (36) final mixtures needed for this purpose are prepared from the four stock mixtures of Table II and in accordance with the recipes therefor which are shown in Table III.

In the reducing step of the permanent wave of this invention, the time and temperature are constant at 30° ± 1° C and 18 minutes ± 5 minutes while the concentration and activity rate of the reducing agent are varied in accordance with the type and condition of the hair. The hair stylist first examines the hair to determine its type according to the degree of damage (Bleach, Tinted, Normal and Resistant), then its

9

porosity (fine, medium, and coarse). Once this examination and selection has been made the stylist simply applies the appropriate reductant mixture from Table I. The reductant will have a specified pH, specified thioglycolate concentration and a specified activity rate for that particular subclass of hair. Since time and temperature are constant in all cases, these variables are eliminated.

The Table III recipes indicate the number of grams of each of the four primary stock mixtures which are to be blended together to form a single dose of a final permanent wave mixture for treating each sub-subclass of hair. Thus, for example, as seen in Table III, to treat "RLF" hair, a final mixture is made from 34 grams of the stock mixture in Container No. 1, 33 grams of the mixture in Container No. 2, 21 grams of the mixture in Container No. 3 and 18 grams of the mixture in Container No. 4. The blending together of the delineated gram-weight portions of the stock mixtures to get the desired final mixture can be done manually (from graduated delivery pipettes attached to the outlets of Containers 1 to 4) or more preferably, by computer controlled measuring and delivery devices. Such latter devices can be used to take into consideration any ambient conditions that might otherwise effect the delivery of the desired measured quantities of the primary mixture into the composite final mixture. Each dose of the final mixture will consist of 106 grams or about 100 ml of solution. One or more doses of such final solutions may be needed to treated a client's hair depending on the amount of hair to be treated.

The amount or quantity of lotion to be applied to the client's hair is then controlled by further classifying the hair according to diameter or thickness of the hair (fine, medium, or coarse), length (short, medium, or long) and finally the density or population of the hair (low, medium, or high). These twenty-seven (27) subcategories based on quantity of hair and the volume of lotion to be applied to the hair thus make it possible to have 972 subclasses of hair to be treated with one of thirty-six (36) different formulas modified as to quantity to provide 972 final individualized formulas as shown in Table IV.

## TABLE IV

Amount Of Table I Permanent Wave (In ml)
Applied To Hair Based On Diameter, Length
And Population Of Hair (Starting ml = 100 ml)

| Subclass Of Hair | | Milliliters |
|---|---|---|
| 1. | FSL | 70 |
| 2. | FSM | 80 |
| 3. | FSH | 90 |
| 4. | FML | 85 |
| 5. | FMM | 95 |
| 6. | FMH | 105 |
| 7. | FLL | 100 |
| 8. | FLM | 110 |
| 9. | FLH | 120 |
| 10. | MSL | 75 |
| 11. | MSM | 85 |
| 12. | MSH | 95 |
| 13. | MML | 90 |
| 14. | MMM | 100 |
| 15. | MMH | 110 |
| 16. | MLL | 105 |
| 17. | MLM | 115 |
| 18. | MLH | 125 |
| 19. | CSL | 80 |
| 20. | CSM | 90 |
| 21. | CSH | 100 |
| 22. | CML | 95 |
| 23. | CMM | 105 |
| 24. | CMH | 115 |
| 25. | CLL | 110 |
| 26. | CLM | 120 |
| 27. | CLH | 130 |

Prior to treating the hair with the permanent wave designated therefor, the hair is washed or otherwise treated to remove any contaminants therefrom that might otherwise interfere with the cold permanent waving process. Such contaminants would include dust, dirt, skin scales, sebum, and residues from hair sprays and conditioners. The hair is then blocked and rolled on permanent wave rods using only water.

The permanent wave is then applied to the hair at a temperature of about $30° \pm 1°$ C. The final mixture of permanent wave is allowed to be in contact with the hair for a period of about 15 to 20 minutes. Subsequently, the thus treated hair is rinsed to remove residues of the final solution and then treated with a neutralizing oxidizing agent to finalize the setting of the hair, using known oxidizing agents such as 2 to 4% hydrogen peroxide.

While this invention has been described by reference to specific examples, it is understood to be limited only by the scope of the appended claims.

## Claims

1. A method for providing permanent waves under constant time and temperature conditions while varying the concentration, pH and activity rate of the reducing agent in accordance with the specific condition of the hair which comprises:

providing a plurality of stock mixtures of reducing agent, each mixture being contained in individual containers having a discreet amount of the mixture;

providing a plurality of sets of criteria for mixing said stock mixtures in accordance with said criteria;

evaluating the hair to choose one set of criteria from said plurality of sets of criteria;

mixing a permanent wave formula from said stock mixtures in accordance with said criteria;

applying said permanent wave to the hair at a temperature of $30° ± 1°C$ for 18 minutes $± 5$ minutes, and

keeping the time and temperature constant for the application of all permanent wave formulas to the differing types of hair.

2. The method of Claim 1 wherein 3-5 stock mixtures are used.

3. The method of Claim 1 wherein 4 stock mixtures are used.

4. The method of Claim 1 wherein agents that are chemically active or time-sensitive are separated among the plurality of stock mixture, said agents being applied to the hair within thirty (30) minutes of mixing the permanent wave.

5. The method of Claim 1 wherein the final mixtures of permanent wave conform to Table I.

6. The method of Claim 1 wherein the stock mixtures conform to Table II.

7. The method of Claim 1 wherein the evaluation to determine concentration, pH and activity rate of the permanent wave formula conforms to Table III.

8. The method of Claim 1 wherein the evaluation to determine the volume of the permanent wave conforms to Table IV.

9. The method of Claim 1 wherein the evaluation of the hair involves four primary classifications of Bleached, Tinted, Normal and Resistant.

10. The method of Claim 9 wherein the hair is further evaluated into nine secondary classes of hair depending on the porosity and diameter of the hair, each of said secondary subclasses being common to each of said primary classes of hair to thereby delineate said hair as one of thirty-six (36) subclassifications thereof.

11. The method of Claim 10 wherein the thirty-six (36) subclassifications are delineated by concentration, pH, and activity rate in accordance with Table III.

12. The method of Claim 11 wherein the hair is treated with permanent wave in accordance with Table I.

13. The method of Claim 12 wherein the hair is further evaluated by quantity of the hair into twenty-seven (27) further subclassifications depending on the diameter, length and population of the hair, each of said subclasses being common to each of said primary classes of hair to thereby delineate said hair as one of 972 subclasses and thereby determine the volume of permanent wave to be applied to the hair in accordance with Table IV.

14. The method of Claim 13 wherein volume of permanent wave conforms to Table IV.

15. A kit for providing permanent waves under constant time and temperature conditions while varying the concentration, pH and activity rate of the reducing agent in accordance with the type and condition of hair being treated which comprises:

a plurality of containers, each of said containers containing mutually compatible stock mixtures of reducing agent;

a plurality of sets of criteria for evaluating the hair according to its condition;

means for evaluating the hair to choose one set of criteria from said plurality of sets;

means for mixing said stock mixtures in accordance with said criteria to form a final mixture of permanent wave; and

means for applying said permanent wave to the hair under constant time and temperature conditions.

12

**16.** The kit of Claim 15 wherein at least one of the plurality of stock mixtures contains a chemically active substance, said substance being kept chemically inactive by separating said substance from a chemically reactive substance prior to being mixed to form the final permanent wave mixture.

**17.** The kit of Claim 15 wherein the means for applying the permanent wave to the hair keeps the temperature at 30° ± 1°C for 18 minutes ± 5 minutes.

**18.** The kit of Claim 17 wherein the plurality of containers conform to Table II.

**19.** The kit of Claim 18 wherein the plurality of sets of criteria conform to Table III.

**20.** The kit of Claim 19 wherein the final permanent wave solution is taken from Table I.

**21.** The kit of Claim 20 wherein the final permanent wave changes as to volume in accordance with Table IV.

**22.** A method for providing tailor-made cosmetic lotions at the point-of-use in accordance with the individual needs and criteria of the client which comprises:
  a plurality of base cosmetic mixtures,
  a plurality of sets of criteria based on differing cosmetic requirements, one set of criteria for each lotion to be mixed,
  selecting one set of criteria upon evaluation of the client's needs,
  mixing the desired cosmetic lotion at the point-of-use in accordance with said criteria, and
  applying said lotion to the client within thirty (30) minutes of mixing the lotion.

**23.** The method of Claim 22 wherein 3-5 base cosmetic mixtures are used.

**24.** The method of Claim 23 wherein said plurality of sets of criteria are compared against statistical averages of said differing cosmetic requirements and preparing a plurality of base cosmetic mixtures taking into consideration said statistical averages such that each base cosmetic mixture is consumed or exhausted at substantially the same rate.

**25.** The method of Claim 24 wherein the base mixtures contain chemically active ingredients, said ingredients being separated among the plurality of base mixtures until said solutions are mixed to thereby avoid any chemical interaction and shelf-stability problems.

**26.** The method of Claim 25 wherein the base mixtures contain anhydrous or water-sensitive ingredients, said ingredients being separated until said solutions are mixed with a water-containing cosmetic lotion.

**27.** A method for providing permanent waves under constant time and temperature conditions of 30° ± 1°C, and 18 minutes, ± 5 minutes, while varying the concentration, pH and activity rate of the reductant in accordance with the specific type and condition of the hair which comprises:
  providing at least three (3) stock mixtures of reducing agent, each of said mixtures containing 2 to 38% reducing agent, the mixture with the least reducing agent also containing 25 to 30% buffer, the mixture with the most concentration of reducing agent also containing 12 to 18% alkali, and the mixture containing no buffer and no alkali containing 1 to 3% of hair conditioner,
  providing a plurality of sets of criteria for mixing said stock mixtures into final reducing agent mixtures of different concentration, pH and activity rate,
  evaluating the hair to choose a single set of criteria from said plurality of sets of criteria,
  mixing a final mixture of reducing agent in accordance with said criteria so that for hair that is most damaged a concentration of reducing agent, buffer and alkali is about 14%, 3% and 5% respectively and the pH is 9.5 to 10.0 and for hair that is the least damaged the concentration is 5.5%, 14%, and 1% respectively and the pH is 6.5 to 7.0, and
  applying said final mixture of reducing agent to the hair.

**28.** The method of Claim 27 wherein the reducing agent is ammonium thioglycolate.

**29.** The method of Claim 28 wherein the buffer is diammonium dithoglycolate.

**30.** The method of Claim 29 wherein the alkali is ammonium hydroxide.

**31.** The method of Claim 30 wherein each stock mixture contains only one color and/or opacifier, mixing said base solutions, and a visible color change occurs upon an erroneous mixing of the secondary solution of permanent wave.

**32.** The method of Claim 30 wherein four (4) stock mixtures are used in four (4) separate containers, and the plurality of sets of criteria are compared against statistical averages of said differing permanent wave requirements, and preparing said four (4) stock mixtures taking into consideration said statistical averages so that the stock mixtures being dispensed and exhausted at substantially the same rate thus avoiding any waste of the stock mixtures.